# EUROPEAN PATENT APPLICATION

(11) **EP 0 965 314 A1**
(43) Date of publication of application: **22.12.1999**
(21) Application number: 98111373.1
(22) Date of filing: 19.06.1998
(51) Int. Cl.: A61F 6/04

(54) **Device for the prevention of sexually transmitted diseases**

(71) Applicant: Makki, Muhammad Fasihuddin, Pune 411008, Maharashtra State (IN)
(72) Inventor: Makki, Muhammad Fasihuddin, Pune 411008, Maharashtra State (IN)
(74) Representative: Hano, Christian, Dipl.-Ing.

(57) **Abstract**

A device to prevent incidence of sexually transmitted disease consists of a multilayered apron (10) sized to cover the pubic area of a user and having a slit or a hole (14) in the middle portion. The apron (10) may take a variety of shapes such as round, rectangular, oval, diamond shaped or the like, and comprises three layers (4, 6, 8) of different materials. The two outer layers (6, 8) consist of absorbent material, such as cotton or other fabric, for absorption of fluids and comfort. The inner layer (4) is made of an impermeable material, such as latex or polythene sheet. The apron (10) is worn over an erect penis fitted with a condom by passing the penis through the slit or hole (14) provided in the apron (10).

## Description

### BACKGROUND OF THE INVENTION

### Field of the Invention

The present invention relates to a device to prevent the incidence of sexually transmitted diseases by contact and/or sexual activity. In particular, the invention relates to a protective barrier interposed between sex partners.

### Description of the Prior Art

The incidence of AIDS is spreading worldwide beyond control. As a result, it has created great fear in developed as well as developing countries. In developed countries, where literacy is high, people tend to exercise more caution in matters of sex. Casual sex has become less fashionable and people are more health conscious. As a result of the stigma and ostracism that some AIDS infected persons have faced in these societies, people have come to consider AIDS with phobic abhor. The incidence of other venereal diseases also pose a potentially great danger. Therefore, the threat is slowly being curbed by education and fear.

In many less developed countries of Africa and Asia, on the other hand, people are still not fully aware of this potentially disastrous situation. It is there that AIDS is likely to spread with epidemic proportions. In many of these countries, war, famine, political upheavals, illiteracy and ignorance undermine sustained efforts by governmental agencies to fight the spread of AIDS. According to recent estimates, India will have the largest incidence of HIV-positive cases among all countries of the world by the year 2000.

It is widely believed that sexual intercourse with a condom does not guarantee safety because of the possibility of condom rupture. In fact, the incidence of condom failure is only about 1 in 1,000,000, but no one has ever thought about the unsuitability of condoms to prevent body fluids or genital secretions from coming into contact with the body of sex partners. During intercourse, wet or moist pubic areas of both participants come constantly into contact. Vaginal secretions can flow out even when a condom is used and come into contact with the pubic area of the condom wearer. These secretions can be dangerous if the person is infected with HIV or any other sexually transmitted disease: The same holds true for a female partner. Even though precautions may have been taken by the male by wearing a condom, his wet moist pubic area is bound to closely contact the female pubic area; therefore, mere use of a condom does not guarantee absolute safety, cleanliness and worry-free exposure for a female as well.

Therefore, there still exists a need for a protective device that further insulates each sex partner from the fluids secreted by the other. This invention is directed at providing such protection through the interposition of a protective apron.

### BRIEF SUMMARY OF THE INVENTION

One object of the present invention, therefore, aims at devising a multilayered apron-like device worn by a male participant to prevent the direct contact of pubic area of the male participant with the pubic area of the female participant.

According to another object of the invention, the device consists of a disposable, simple, shield-like or apron-like curtain or barrier that prevents the pubic areas to come into direct contact. In particular, the main goal is directed at preventing any exposed vaginal secretions to come into direct contact with the male pubic area.

Therefore, according to these and other objectives, the present invention consists of a multi-layer apron having a slit or a hole in the middle portion. The apron may take a variety of shapes such as round, rectangular, oval, diamond shaped or the like, and comprises three layers of different materials. The two outer layers consist of absorbent material, such as cotton or other fabric, for absorption of fluids and comfort; while the inner layer is made of an impermeable material, such as latex or polythene sheet.

The apron can be worn over an erect penis fitted with a condom by passing the penis through the slit or hole provided in the apron.

In a another embodiment of the invention the multilayered sheet structure is integral with a condom, the opening of the sheet structure forming the lead-in opening of the condom.

Various other purposes and advantages of the invention will become clear from its description in the specification that follows and from the novel features particularly pointed out in the appended claims. Therefore, to the accomplishment of the objectives described above, this invention consists of the features hereinafter illustrated in the drawings, fully described in the detailed description of the preferred embodiment and particularly pointed out in the claims. However, such drawings and description disclose but one of the various ways in which the invention may be practiced.

### BRIEF DESCRIPTION OF TNE DRAWINGS

Figs. 1-5 are plan views of various embodiments of aprons according to the present invention.

Fig. 6 illustrates the multilayer construction of the composite material used for the invention.

Fig. 7 is a plan view of the preferred embodiment of the invention.

Fig. 8 shows a further embodiment of the apron according the invention.

### DESCRIPTION OF TNE PREFERRED EMBODIMENTS OF TNE INVENTION

This invention utilizes the idea of combining a layer of impermeable material with two layers of absorbent material on the sides thereof to absorb fluids released by either partner during sexual intercourse and prevent their passage to the other partner. The invention will now be described with the help of the drawings accompanying this specification, wherein like parts are designated throughout with like numerals and symbols.

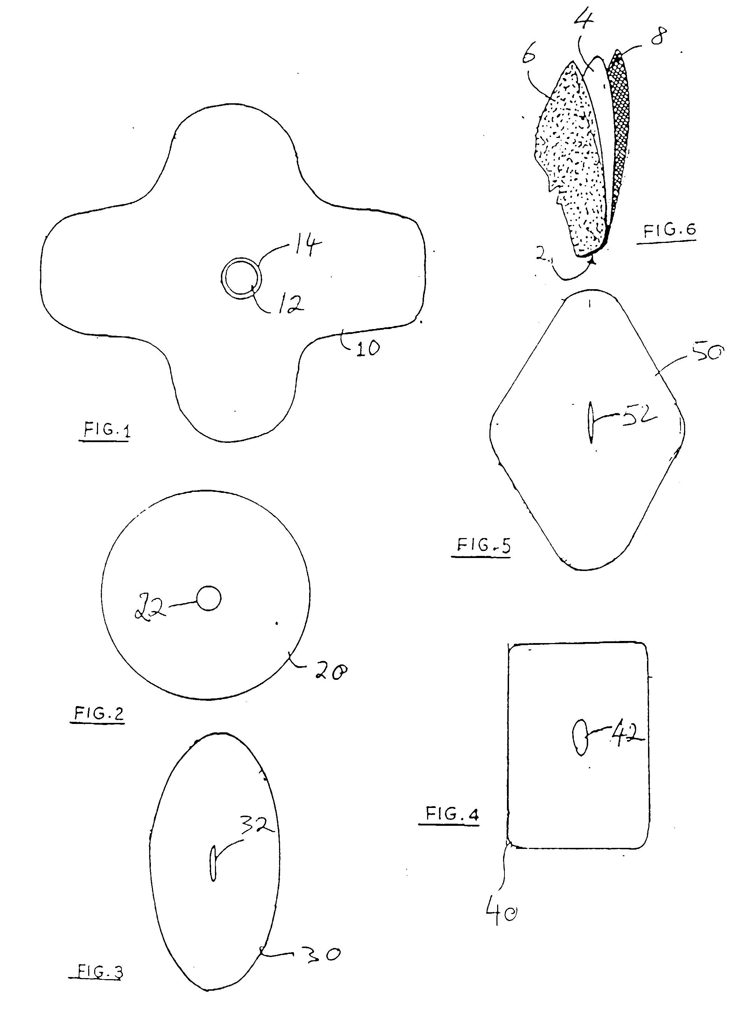

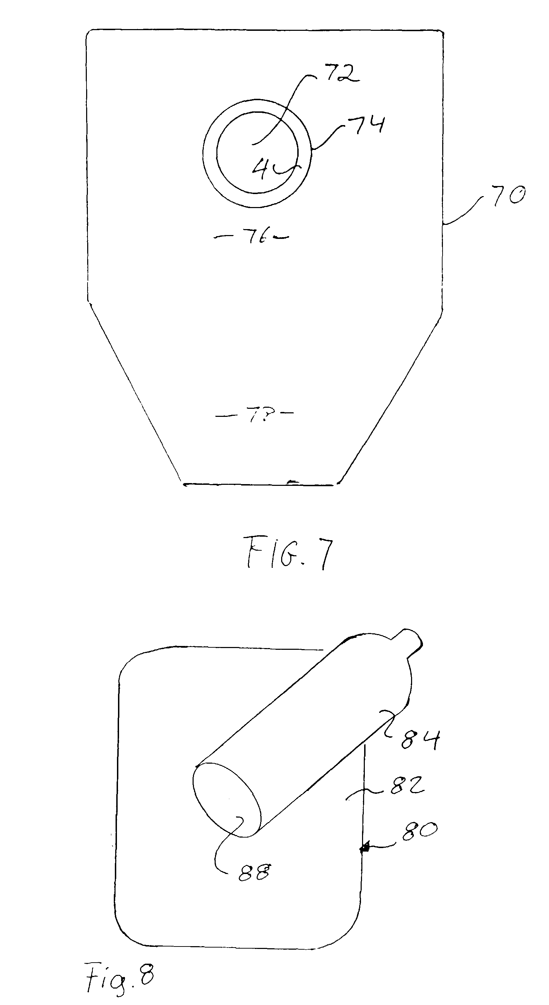

Figs. 1-5 show various embodiments of the invention with different shapes and sizes. In all these figures, an apron is illustrated to prevent transmission of sexually transmitted disease by contact and/or by contamination by way of sexual activity. All embodiment consists of a multilayered sheet composite 2, shown in Fig. 6, wherein an inner layer 4 is a flexible, stretchable and impermeable material such as polythene or latex, while two outer layers 6 and 8 are fabric or other absorbent material, such as cotton or silk, adhered to the inner layer 4. Layers 6 and 8 are illustrated as consisting of different materials, but they can equivalently be constructed of the same absorbent material. All embodiments of Figs. 1-5 are made with sheets 2 of the multilayered composite shown in Fig. 6 and are appropriately sized to cover the pubic area of a user.

Referring to Fig. 1, a star-shaped embodiment 10 is illustrated wherein a hole 12 is provided in the sheet composite 2 so that it can be placed over the erected penis of a user prior to intercourse. A narrower hole 14 of appropriate diameter in the stretchable inner layer 4 may be provided to form a collar ring for a firmer, snug connection with the penis.

The apron 20 in Fig. 2 illustrates a circular construction and an opening 22 of equal diameter for all three layers 6, 4 and 8. Fig. 3 illustrates an oval shape apron 30 with an opening consisting of a slit 32. Because all three layers 6, 4 and 8 constituting the sheet composite 2 are stretchable, the slit 32 can be used equivalently to the hole 12 for snugly placing the apron of the invention around the penis of a user. Figs. 4 and 5 show rectangular and rhomboid shapes, respectively, with slits 42 and 52 shaped differently for illustration.

The use of the apron of the present invention is very simple. The first layer 6 of fabric is facing the body, for comfort. The middle layer 4 of impermeable film is meant to prevent any body fluid from a partner to come into contact with the pubic area of the user. The third layer 8 is also fabric or other suitable absorbent material to catch fluids released from the partner. This layer is meant to absorb moisture, sweat, or any other bodily fluids and prevent them from spreading and smearing, or touching the pubic area of either male or female. The collar ring 14 around the hole 12 is meant to fit over a condom worn by a user to effectively prevent the passage of any body fluids exposed or seeped from a partner from coming into contact with the pubic area of the condom wearer.

Fig. 7 shows the preferred embodiment of the invention, wherein an apron 70 is shaped substantially like a bib. The sheet composite 2 consists of an inner latex layer 4 and cotton outer layers 6 and 8. The opening 72 is circular and provides a collar formed in the inner layer 4 of latex, surrounded by a preferably concentric larger circular hole 74 in the outer layers 6 and 8 of cotton. The apron 70 is approximately 16 cm high, with an upper substantially square portion 76 having 12 cm sides and a lower portion 78 with a 6-cm lower side. The opening 72 in the latex layer 4 is about 2.5 cm in diameter and the opening 74 in the outer layers is about 4 cm in diameter. A slit about 2.5 cm long through all three layers 6,4,8 could be used equivalently.

The apron 80 shown in Fig. 8 comprises a condom 84 which is integral with the sheet composite 82. The condom 84 forms one part with the impermeable layer of the sheet composite 82. The opening 88 of the sheet composite forms the lead-in opening of the condom 84.

Various changes in the details, steps and components that have been described may be made by those skilled in the art within the principles and scope of the invention herein illustrated and defined in the appended claims. Therefore, while the present invention has been shown and described herein in what is believed to be the most practical and preferred embodiments, it is recognized that departures can be made therefrom within the scope of the invention, which is not to be limited to the details disclosed herein but is to be accorded the full scope of the claims so as to embrace any and all equivalent devices.

## Claims

1. An apron to prevent transmission of sexually transmitted diseases comprising, in combination:
- a multilayered sheet structure (2, 10, 20, 30, 40, 50, 70, 82) comprising an inner layer (4) of stretchable material and two outer layers (6, 8) of absorbent material, said structure (2, 10, 20, 30, 40, 50, 70, 82) being appropriately sized to cover a pubic area of a user;
- an opening (12, 22, 32, 42, 52, 72, 88) in said sheet structure (2, 10, 20, 30, 40, 50, 70, 82) adapted to receive and snugly connect the sheet structure (2, 10, 20, 30, 40, 50, 70, 82) to a penis of the user.

2. The apron of Claim 1, wherein said inner layer (4) is made of latex or polythene.

3. The apron of Claim 1 or 2, wherein said outer layers (6, 8) are made of cotton or silk.

4. The apron of Claim 1 or 2, wherein one of said outer layers (6, 8) is made of cotton and the other is made of silk.

5. The apron of one of the preceding Claims, wherein said opening consists of a circle about 4 cm in diameter.

6. The apron of one of the claims 1 to, wherein said opening consists of a slit about 4 cm long.

7. The apron of Claim 1, wherein said opening comprises an orifice (72) in said inner layer (4) and concentric larger apertures (74) in the outer layers (6, 8) forming a stretchable collar around the opening.

8. The apron of Claim 7, wherein said orifice (72) is about 2.5 cm in diameter and said apertures (74) are about 4 cm in diameter.

9. The apron according to Claim 1, wherein the multilayered sheet structure (82) is integral with a condom (84), the opening in the sheet structure (82) forming the lead-in opening of the condom (84).

10. An apron to prevent transmission of sexually transmitted diseases comprising
a impermeable sheet structure (82) being appropriately sized to cover a pubic area of a user,
an opening (88) in said sheet structure (82) adapted to receive and snugly connect the sheet structure (82) to a penis of the user,
the sheet structure (82) being integral wih a condom (84), the opening (88) in the sheet structure (82) forming the lead-in opening of the condom (84).
